# EUROPEAN PATENT SPECIFICATION

(11) **EP 1 108 433 B1**
(45) Date of publication and mention of the grant of the patent: **20.10.2004**
(21) Application number: 00127372.1
(22) Date of filing: 13.12.2000
(51) Int. Cl.: A61K 39/10, A61K 39/40, A61P 31/04

(54) **Combination vaccine for enhancing immunity against brucellosis**
Kombinationsimpfstoff zur steigerung der Immunität gegen Brucellose
Vaccin combiné pour augmenter l'immunité contre la brucellose

(30) Priority: 15.12.1999 US 170765
(43) Date of publication of application: 20.06.2001
(73) Proprietor: THE MINISTER OF NATIONAL DEFENCE OF HER MAJESTY'S CANADIAN GOVERNMENT, Ottawa Ontario, K1A 0K2 (CA)
(72) Inventor: Cherwonogrodzky, John W., Medicine Hat, Alberta T1B 3L5 (CA)
(74) Representative: Goddar, Heinz J., Dr.

(56) References cited:
- WO-A-93/16728
- US-A- 5 951 987
- ALONSO-URMENETA B ET AL: "Evaluation of lipopolysaccharides and polysaccharides of different epitopic structures in the indirect enzyme-linked immunosorbent assay for diagnosis of brucellosis in small ruminants and cattle." CLINICAL AND DIAGNOSTIC LABORATORY IMMUNOLOGY, vol. 5, no. 6, November 1998 (1998-11), pages 749-754, XP002239483 ISSN: 1071-412X
- VIZCAINO NIEVES ET AL: "Molecular characterization of a Brucella species large DNA fragment deleted in Brucella abortus strains: Evidence for a locus involved in the synthesis of a polysaccharide." INFECTION AND IMMUNITY, vol. 67, no. 6, June 1999 (1999-06), pages 2700-2712, XP002239484 ISSN: 0019-9567

## Description

### FIELD OF INVENTION

The present invention relates to a vaccine, comprising a combination of bacterial components derived either from different species of *Brucellae,* or one strain expressing different components, that enhances immunity against brucellosis. The vaccine formulations are applicable for one or more cross-reactive bacteria thereof.

### BACKGROUND OF THE INVENTION

Brucellosis is a debilitating disease that can cause abortions and weight loss in animals as well as undulating fevers, night sweats, incapacitation and arthritis in humans. It is very hardy to environmental factors, easily aerosolized and infectious through skin abrasions, ingestion and the pulmonary route. It is difficult to treat with antibiotics and often persists as a life-long infection. Brucellosis is a disease endemic to most countries, especially underdeveloped nations where *Brucella* species infect 0.1 to 10% of the livestock such as cattle, swine, sheep, goats, and camels. A zoonotic disease, these also infect other domestic animals such as dogs and poultry, wildlife such as bison, caribou and wolves and marine mammals such as whales and dolphins. People are especially vulnerable to infection either through handling infected products or ingesting contaminated foods.

Up-to-date, effective treatment against brucellosis for animals, including humans, has been limited. For humans, administering high doses of combination antibiotics, for example doxycycline with rifampin over long periods, has been found to be effective to clear the disease, but non-compliance and relapses are common. For animals, the cost and limited effectiveness of antibiotic treatments often lead to the decision of either no treatment or elimination of the infected animal and its associated herd.

The most preferred type of disease management is to avoid infection and to reduce the incidence and spread of the disease by vaccination. For livestock, namely cattle, at present vaccination consists of using an attenuated (weakened) vaccine strain such as *Brucella abortus* strain 19. Although it is one of the best vaccines for cattle, it does have limitations in that the vaccine does not give absolute protection and there is about a 20% failure rate, results from serological tests can be confusing for a positive serology may be caused by vaccination, infection, or vaccination with subsequent infection, the vaccine although tolerated by cattle is pathogenic for humans, and on occasion the vaccine does revert to a "wild" or virulent form.

For humans, there existed a French vaccine that consisted of a phenol insoluble residue. However, this vaccine has been discontinued as it was found that the residue caused a high rate of reactogenicity (in one study, a large percentage of the vaccine recipients developed swollen lymph glands and granuloma at the site of injection) and hyper-sensitivity (vaccinates that touched killed *Brucella* preparations presented symptoms of anaphylactic shock).

Recently, the Applicant has discovered a new vaccine that protected animals (e.g. mice, guinea pigs and swine) from brucellosis and which may upon further development be suitable for protecting humans. The vaccine is as described in US Patent No. 5,951,987. The vaccine consists of an outer-polysaccharide (OPS) isolated from *Brucella* such as *Brucella abortus.* The vaccine protected animals from different strains and species of *Brucella* tested (e.g. *B. abortus* 30, *B. abortus* 2308 and *B. suis* biovar 1) as well as infections from *Francisella tularensis* live vaccine strain (LVS) which causes tularemia in mice. This gave evidence that the vaccine would likely offer effective protection against infections from a broad spectrum of *Brucella* species and cross-reactive bacteria. However, it has subsequently been found otherwise. Although the *B. abortus* OPS vaccine was effective in offering animals protection from brucellosis, it did so only against species and strains that resembled *B. abortus* in serology (i.e. had "A" OPS antigens). It did not appear to be effective against species and strains that resembled *B. melitensis* in serology (i.e. had the "M" or "A&M" OPS antigens). Hence, there still remains a need for a vaccine which is effective against infections from a wide spectrum of *Brucella* species.

### SUMMARY OF THE INVENTION

In accordance with one aspect of the present invention, there is provided a vaccine comprising a combination of *Brucella* "A" and "M" outer-polysaccharides and "R" protein antigens. The outer-polysaccharides may be obtained from the same or different species of *Brucellae.* The most preferred source of OPS is derived from *Brucella* but a logical extension of this finding is to use bacterial species cross-reactive thereof.

The combination may be obtained from combining "A" outer-polysaccharides extracted from *Brucella* species selected from the group consisting of *B. abortus* biovar 1, *B. abortus* biovar 2, *B. abortus* biovar 3, *B. abortus* biovar 6, *B. melitensis* biovar 2, *B. suis* biovar 1, *B. suis* biovar *2, B. suis* biovar 3, *B. neotomae* and *B. maris;* "M" outer-polysaccharide extracted from *Brucella* species selected from the group consisting of *B. abortus* biovar 4, *B. abortus* biovar 5, *B. abortus* biovar 9, *B. melitensis* biovar 1, *B. suis* biovar 5; and "R" core polysaccharide and proteins extracted from *Brucella* species selected from the group consisting of *B*. *ovis* and *B. canis.*

Alternatively, the combination may be obtained by combining "AM" outer-polysaccharides extracted from *Brucella* species selected from the group consisting of *B. abortus* biovar *7, B. melitensis* biovar 3 and *B. suis* biovar 4 (note: *B. suis* 145 biovar 4 is used in the present patent submission), and "R" core polysaccharide and protein extracted from *Brucella* species selected from the group consisting of *B. ovis* and *B. canis.*

In accordance with another aspect of the present invention, there is provided a vaccine comprising a combination of *Brucella* outer-polysaccharides containing the "A" and "M" antigens and a *Brucella* outer-polysaccharide-protein complex.

In this case, the combination may be obtained by combining "A" outer-polysaccharide purified from *Brucella* species selected from the group consisting of *B. abortus* biovar 1, *B. abortus* biovar 2, *B. abortus* biovar 3, *B. abortus* biovar 6, *B. melitensis* biovar 2, *B. suis* biovar 1, *B. suis* biovar 2, *B. suis* biovar 3, *B. neotomae* and *B. maris;* "M" outer-polysaccharide purified from *Brucella* species selected from the group consisting of *B. abortus* biovar 4, *B. abortus* biovar 5, *B. abortus* biovar 9, *B. melitensis* biovar 1, *B. suis* biovar 5; and an outer-polysaccharide-protein complex selected from the group consisting of outer-polysaccharide and *Brucella* membrane proteins, outer-polysaccharide and *Brucella* surface proteins, outer-polysaccharide and *Brucella* surface enzymes and outer-polysaccharide and *Brucella* cytoplasmic proteins.

Alternatively, the vaccine may be obtained by combining "AM" outer-polysaccharides extracted from *Brucella* species selected from the group consisting of *B. abortus* biovar 7, *B. melitensis* biovar 3 and *B. suis* biovar 4, and an outer-polysaccharide having a protein selected from the group consisting of *Brucella* membrane proteins, *Brucella* surface proteins, *Brucella* surface enzymes and *Brucella* cytoplasmic proteins.

The vaccine may consist of 1 ng to 100ug, preferably lug, of each of the OPS forming the combination for vaccination of mice weighing about 20 grams.

The vaccine is effective as a prophylactic treatment from infection against a wide range of *Brucella* species namely *B. abortus, B. melitensis* and *B. suis.* By logical extension the vaccine is likely to be effective for the prevention of brucellosis from *B. ovis, B. canis, B. neotomae* and *B. maris.* Animal studies support its use as a vaccine for livestock, and with further development possibly as a vaccine for humans. It is most effective by intra-peritoneal, sub-cutaneous and intra-muscular administration. It is least effective when given intranasally. The vaccine works best against the most virulent species and strains of *Brucella,* most of the healthy vaccinates having no bacteria in their spleens or having a million fold less bacteria than controls. The vaccine works, but is less effective where it is not needed, or in mice given *Brucella* species and strains of low virulence.

Serum or white blood cells of mammals vaccinated with the vaccine in accordance with the present invention prevented brucellosis in recipient animals. Protection is long term (i.e. at least several weeks) but unlike other vaccines it is also protective in the short term (i.e. protective in 1 day or less).

### DETAILED DESCRIPTION OF THE INVENTION

*Brucella* species can be classified by their different type of outer-polysaccharides (OPS). These serological types are those having the "A" OPS, the "M" OPS and those lakking OPS or the "R" group (i.e. antigens are predominantly protein with some antigenicity being the "core" polysaccharides attached to the lipopolysaccharide, or LPS, lacking OPS), wherein "A", "M" and "R" stands for the type of antigens. Some species also express more than one antigen, for example some strains of *B. suis* (biovar 4) which express both the "A" and "M" antigens, and some are capable of changing their antigens within the same host. The various types of OPS are very similar in chemical structures. They are generally made of identical sugars, which are linked differently. Because of the similarity between the OPS and the considerable cross-reaction between the "A" and "M" OPS of *Brucella,* one would expect a single OPS vaccine, i.e. a vaccine consisting of one type of OPS, to be effective against a wide range of *Brucella* species. This, however, was found to be only partially true. The Applicant discovered that a combination or "cocktail" vaccine, i.e. a vaccine having a combination of the different OPS, is much more effective than the single OPS vaccine in providing protection against a wide spectrum of *Brucella* species.

To be most effective, the cocktail or combination vaccine should include a range of OPS, for example both "A" and "M" OPS, and "R" antigens. As it is likely that one or more OPS, other than "A" and "M", are also produced (Vizcaino, N., Cloeckaert, A., Zygmunt, M.S., and Fernandez-Lago, L. 1999. Molecular characterization of a *Brucella* species large DNA fragment deleted in *Brucella abortus* strains: evidence for a locus involved in the synthesis of a polysaccharide. *Infection and Immunity,* 67: 2700-2712), the inclusion of these would likely offer greater protection.

The cocktail or combination vaccine may be replaced by an outer-polysaccharide-protein complex. *Brucellae* can attach proteins (the "R" antigen, which can also be extracted from cells that do not express the "A" or "M" antigen, have colonies rough in appearance and express mainly proteins) to OPS. OPS and OPS-protein complex can be separated (e.g. with 0.2 M trichloroacetic acid, OPS remains soluble, OPS-protein precipitates).

Outer-polysaccharides containing the "A" antigen can be obtained from *B. abortus* biovar 1, *B. abortus* biovar 2, *B. abortus* biovar 3, *B. abortus* biovar 6, *B. melitensis* biovar 2, *B. suis* biovar 1, *B. suis* biovar 2, *B. suis* biovar 3, *B. neotomae* and *B. maris.*

Outer polysaccharides containing the "M" antigen can be extracted from *B. abortus* biovar 4, *B. abortus* biovar 5, *B. abortus* biovar 9, *B. melitensis* biovar *1, B. suis* biovar 5.

Proteins, core polysaccharides and short chained outer-polysaccharides comprising the "R" antigen can be purified from *B. ovis* and *B. canis.*

Outer-polysaccharides containing both the "A" and "M" antigens can be purified from *B. abortus* biovar *7, B. melitensis* biovar 3 and *B. suis* biovar 4.

Suitable proteins for the "R" component are *Brucella* proteins. Preferably, they are outer-membrane proteins (opm) such as opm1, opm2 and opm3, lipoprotein linked to cell wall, porin (a protein that allows ions or metabolites through the membrane), A5 on the cell surface, surface proteins such as a, b and X, surface enzymes such as protease, Brucellin proteins and internal or cytoplasmic proteins. Other proteins may be mannosyltransferase, GDP-mannose 4,6 dehydratase, perosamine synthetase, ABC-type transporter and formyl transferase. Additional proteins such as those identified in the paper "Conservation in *Brucella* spp. of seven genes involved in the biosynthesis of the lipopolysaccharide O-chain", A. Cloeckaert, M. Grayon, J-M Verger, J-J Letesson and F. Godfroidt, 1998. 51th Annual Brucellosis Meeting, Chicago, can also be used.

To assess the effectiveness of the vaccine, different single OPS vaccine and combination OPS vaccines were prepared and tested. The novel formulation of using combination OPS vaccine of the present invention is shown to be more effective than a single OPS vaccine formerly disclosed by the Applicant in the US Patent No. 5,951,987.

### MATERIALS AND METHODS

### Bacterial cultures:

*B. abortus* 30, *B. abortus* 2308, *B. melitensis* 16M and *B. suis* 145 were acquired in 1989 from the Animal Diseases Research Institute (ADRI-Nepean, now the Canadian Food Inspection Agency), Nepean, Ontario, Canada. The bacteria were thawed and small aliquots of the materials were obtained and grown on *Brucella* agar (Difco Laboratories, Detroit) with 1.5 ppm crystal violet at 37°C, 5% CO₂, 90% humidity, for 1 week. A loopful (about a billion cells) of the culture were placed in vials containing 1 ml of sterile *Brucella* broth with 15% glycerol and the vials were frozen at -70°C. As required, vials containing *B. melitensis* 16M, *B. suis* 145, *B. abortus* 30 and 2308 were thawed and subcultured onto, for example, *Brucella* agar and incubated to provide cells for Protect Beads^{TM} storage, or into *Brucella* broth and incubated to provide cells which were later used in the infectivity experiments.

Representative vials of the bacteria were thawed and used to inoculate *Brucella* agar slants (2 cc agar in a 5 cc vial). The cultures were verified on May 10, 1999 by the National Veterinary Services Laboratories (Ames, Iowa), which confirmed that the vials containing *B. abortus* 30 or *B. abortus* 2308 belonged to *B. abortus* biovar 1 ("A" antigen predominant), *B. melitensis* 16M belonged to *B. melitensis* biovar 1 ("M" antigen predominant), *B. suis* 145 belonged to the atypical *B. suis* biovar 4 (has both "A" and "M" antigens).

The bacteria used to cause *Brucella* infection were prepared by inoculating bacteria such as *B. melitensis* 16M into *Brucella* broth (Difco Laboratories, Detroit), grown overnight, then 0.010 ml was transferred to 100 ml *Brucella* broth and used within 18 hours of incubation. This method revealed more effective in providing bacteria with high virulence than the conventional method of simply thawing a frozen stock of bacteria and determining the colony forming unit (CFU), or adding the thawed stock to prewarmed *Brucella* broth and incubating for 2 hours at 37°C, 5% CO₂ and 90% humidity.

The best way to ensure virulence of the bacterium (e.g. *B. melitensis* 16M) was to passage it through sets of 5 mice. Hence a culture that appeared to have lost its virulence, would be given to each of 5 mice by intra-peritoneal injection. In this case, each mouse was given 5 × 10⁴ to 5 × 10⁵ bacteria/0.1 ml sterile saline. After 1 week, the mice were sacrificed, their spleens weighed and crushed in saline, and serially diluted and plated on *Brucella* agar with 1.5 ppm crystal violet and incubated for 2 hours at 37°C, 5% CO₂ and 90% humidity and the colony forming unit was determined. Bacteria on these plates that were from the mouse with the largest spleen size and greatest bacterial number in the spleen was selected and these were used to infect another 5 mice and the selection was repeated. Within 3 passages, the bacterium had exceptional virulence (caused large spleens, 3-5 fold larger than normal, and high bacterial numbers, about 3 million bacteria per spleen). These bacteria were used only for a few experiments as it was viewed that passage through an animal could introduce other unknown variables.

### Vaccine preparation:

### a) Preparation of "A" OPS, "M" OPS or OPS-protein vaccines

The method of preparation for some of the vaccines (e.g. "A" OPS from *B. abortus* 1119-3, "M" OPS from *B. melitensis* 16M) was as previously described ("Antigens of *Brucella", J.* W. Cherwonogrodzky et al., pages 54-55, In: K. Nielsen, J.R. Duncan (ed.) 1990 Animal Brucellosis CRC Press, Boca Raton). Briefly, each bacterium was grown on about 90 ml of *Brucella* agar with 1.5 ppm crystal violet into each of twenty 150cm² sterile tissue culture flasks and incubated for 2 hours at 37°C, 5% CO₂ and 90% humidity. After the incubation period, 5 ml of sterile 3% acetic acid with 1% saline was added to each flask, a dozen small glass beads were added, and the cells made into a suspension by rocking or lightly shaking the flask as required. The suspension was removed with a pipette and placed into a 250 ml centrifuge bottle. Another 5 ml of sterile acetic acid with saline in triply distilled water was added to the flask. The flask was rocked or shaken as required, and the suspension was added to the previous one.

Once all twenty flasks were processed, the suspension was shaken vigorously, and stored at 4°C for at least one week. Thereafter, it was autoclaved at 121°C, 15 psi for 2 hours with a loosened cap. Once autoclaved and cooled, the bottle was centrifuged at 15,000 X g for 30 min at 4°C. The supernatant was kept and the cells discarded.

The supernatant was neutralized with 10 M NaOH and had 2 M trichloroacetic acid (TCA) added to a final concentration of 0.2 M TCA. This was centrifuged (20,000 X g, 30 min, 4°C). The OPS remained soluble in the supernatant, the OPS-protein that was precipitated by the TCA was in the pellet. The pellet was resuspended in triply distilled water. Both the supernatant and the redissolved pellet (kept separate) were extracted with equal volumes of liquified phenol (90% phenol with 10%water) added. The mixture was magnetically stirred for 30 min at 70°C and was chilled at 4°C overnight. The bottom phenol layers were removed and centrifuged as before to remove debris or the remaining water layer. To the phenol layers were added 5 volumes of methanol with 1% sodium acetate to enhance precipitation and this was chilled overnight at 4°C. The preparations were centrifuged as before, washed twice more with a similar volume of methanol-acetate, and the pellet was dissolved in triply distilled water and dialyzed (1000 mw cutoff) against triply distilled water. Once dialyzed, these samples were centrifuged to remove denatured material and lipopolysaccharide (LPS, this aggregates in distilled water while outer-polysaccharide (OPS) remains soluble). The samples were freeze-dried, weighed and kept at -70°C until required. The resulting samples were at least 90% pure.

### b) Preparation of B. suis 145 OPS vaccine

When the previous method for OPS preparation was applied to *B. suis* 145, it was unsuccessful. Neither OPS nor OPS-protein precipitated when methanol-acetate was added to the phenol extracts. The material was still present, as evidenced by OPS and OPS-protein precipitating when the phenol/methanol-acetate solutions were kept at -20°C instead of 4°C for a week, but it was clear that the procedure had to be changed to prepare OPS or OPS-protein for vaccines. (It was subsequently found that *B. suis* 145 OPS is more heterogeneous in composition than the other *Brucella* OPS cited, explaining the failure of the previous method, Dr. Brad Berger, unpublished results.)

In brief, *B. suis* 145 was grown on agar medium *(Brucella* agar, with or without 1.5 ppm crystal violet, trypticase soy agar, with or without 1.5 ppm crystal violet, gave similar results) in sterile 150 cm² tissue culture flasks, at 35°C, 5% CO₂ and 90% humidity for 1 week. After this time, cells were both killed and removed by adding 5 ml of 5% phenol/ 1% saline, adding glass beads, and rolling or shaking the flask to dislodge the cells, removing the cells, adding another 5 ml of phenol-saline, rolling and shaking the flask again, and pooling cell suspensions. From 400 flasks, about 250 grams wet weight of cells was removed and the final volume was about 3 litres in sixteen 250 ml centrifuge bottles. The suspension was kept 1 week at 4°C, with occasional shaking, to ensure release of loosely bound antigens.

After 1 week, the suspension was centrifuged (15,000 X g, 20 min, 4°C), the supernatants pooled, and the cells washed with a small volume (40 ml per centrifuge bottle, centrifugation as before) of phenol-saline. The liquid was added to the pooled supernatants.

To the supernatant, glacial acetic acid was added to a final volume of 3%. This suspension was then placed in a boiling water bath for 2 hours. It was left to cool to room temperature for a day. The pH was not adjusted (the low pH appears to enhance precipitation at a later methanol stage). A one-half volume of 90% phenol (10% water) was added (the smaller volume concentrates the OPS). This was magnetically stirred on a magnetic hot-plate until the temperature rose to the mixture's "clarity" point (the phenol-water mixture was opaque, but around 65-70°C the phenol and water dissolved into each other, causing the mixture to clear). It was then allowed to cool at 4°C overnight, centrifuged and the phenol layer (bottom layer, usually dark red in colour) kept. A 2-week sterility check is done to ensure this phenol layer is sterile and it is then taken out of Biocontainment 3. Once in the general laboratory area, the phenol was chilled overnight at 4°C as was the methanol-acetate (methanol with 1 % sodium acetate.3H₂O). Five volumes of methanol-acetate was added to the phenol layer, this was mixed on a magnetic stirrer and allowed to settle for 1-2 days. After this time, most of the liquid above the precipitate was aspirated away (the flask is placed on ice to prevent mixing if it begins warming to room temperature). The remaining preparation was centrifuged as before, the supernatant discarded, and the pellet resuspended in methanol-acetate (a saw-toothed OmniMix^{TM} is best for blending the suspension) and centrifuged. The pellet was then resuspended in distilled water, placed in a 1000 mw cutoff dialysis cellulose membrane, and dialyzed against distilled water at 4°C. (From the frothing that occurred when the outside water was discarded during changes, it appeared that considerable amount of small m.w. OPS was lost in this process, but that a large amount of larger m.w. OPS was retained by the dialysis.) After dialysis, the preparation was removed from the dialysis bag, and centrifuged to remove denatured material. The pellet of denatured material was discarded, the supernatant was kept, and to the latter 2 M trichloroacetic acid (TCA) was added until the final concentration was 0.2 M TCA. This was then centrifuged. Both the supernatant (containing OPS) and the pellet (containing OPS-protein, this was suspended in distilled water) were dialyzed against distilled water. After extensive dialysis, each preparation was centrifuged to remove denatured or particulate material. The solutions were then aliquoted and freeze-dried. The OPS could be further refined with enzyme digestion (though the starting material was at least 90% pure, having no detectable A260/A280nm absorbing nucleic acids and only about 0.6% protein) and ultra-centrifugation (120,000 X g, 4°C, 3 hours) to remove trace amounts of LPS.

For the washed *B. suis* 145 cells noted previously, these were suspended in 3% acetic acid and 1% saline (for every gram of cells, 5 ml of acetic acid-saline was added) and placed in a boiling water bath for 2 hours with swirling to mix the suspension every half hour. The cells were left for a day to cool to room temperature. The preparation was centrifuged, and the supernatant kept. The cells were mixed in an equal amount (w/v) of acetic acid saline, centrifuged, and the liquid pooled with the other. A phenol extraction was done (half a volume of phenol was used with the cell supernatant) on this liquid as noted before. The OPS released from the cell by boiling water was by the method noted above.

The cells from the above were resuspended in 5 volumes of acetic acid-saline, autoclaved (121°C, 15 psi, 2 hours), cooled, centrifuged, and the liquid (as well as a washing of the cells with an equal amount of acetic acid-saline which was added) was processed as before. The different fractions noted above have been tested (1ug/0.1 ml sterile saline/mouse, intraperitoneal injection). All OPS fractions and all OPS-protein fractions were protective (about 10,000 fold less bacteria in their spleens than controls 1 week after challenge). The whole cell with OPS removed, or the interphase material (between the phenol and water layers during extraction) were not protective (indeed, the interphase material was immunosuppressive, causing mice to have about 5-fold more bacteria in their spleens than non-vaccinated control mice).

About 4-fold more OPS was acquired if the *B. suis* 145, was passaged through a mouse before being grown on agar. There was also a considerable amount of brown gelatinous material on the cells after autoclaving (about 20 c.c. on 250 grams of cells, when 2 cc of this was freeze-dried and gave 80 mg dry weight). However, as passaging through a mouse might be introducing new variables, frozen stocks kept on ProtectBeads^{TM} at -70°C were used as the inoculum for OPS and OPS-protein production.

The strength for using *B. suis* 145 as a source of vaccine is that as it expresses both "A" and "M" OPS (as well as *Brucella* "R" proteins) it is also likely to express other OPS. Recently another OPS has been isolated, by a method unobvious to anyone skilled in the art. There was an insufficient amount of this polysaccharide to characterize, other than it was polysaccharide, but animal studies showed that this OPS (which is in the OPS vaccine preparation) also protects mice from brucellosis.

As stated previously in this text, the *B. suis* 145 OPS or OPS-protein preparations were found to be potent vaccines that protected mice from brucellosis. Against the most virulent strains and species of *Brucella,* vaccinated mice often had no bacteria in their spleens, or only a few (i.e. a million fold less than controls). For the latter, it was unknown if these were simply the last traces of the challenge that were soon to be cleared (which is likely for their growth lag on agar medium suggests that these are heavily damaged by the immune system of the host) or whether these were mutants with polysaccharides different from what the mice were vaccinated against. These "survivors" were allowed to continue growing on the agar plates previously used to assess spleen bacterial loads (35°C, 5% CO₂, 90% humidity, for an additional 3 weeks), these were scraped from the plates and suspended (3.3 grams wet weight of cells was recovered) in 5% phenol, 1% saline (50 ml) and processed as noted above. Mice have been vaccinated with *B. suis* 145 OPS (as noted above, 1 ug/mouse by intraperitoneal injection), or OPS prepared from *B. suis* 145 bacterial "survivors" from vaccinated mice that were challenged (1 ug/mouse by i.p.), or *B suis* 145 OPS + *B. suis* 145 "survivors" OPS (both 1 ug/mouse by i.p.). This study is underway but at the time of this patent submission it has not determined if the OPS from bacterial "survivors" is equivalent to *B. suis* 145 OPS for protecting mice from brucellosis or whether it acts synergistically to enhance vaccine efficacy.

### Mice:

Unless otherwise specified, mice were 19-21 grams at the start of the experiment. They were female balb/c mice obtained from Charles River, St. Contance, Quebec, Canada.

### Vaccination:

Mice were vaccinated intra-peritoneally (i.p) into the lower right side of the belly, subcutaneously (s.c.) into the nape of skin bunched on the back, intra-muscularly (i.m) into the upper left leg or intra-nasally (i.n.). The intra-nasal route required mice to be anaesthetized with Metofane^{TM}. Generally, the vaccines comprised OPS from *B. abortus* 1119-3, *B. melitensis* 16M and/or *B. suis* 145 in sterile saline as a single dose per mouse, or a combination of OPS and OPS-protein. For example, a single antigen vaccine can be 1 ug of *B. suis* OPS in 0.1 ml sterine saline as single dose per mouse. An example of a combination vaccine can be 1 ug each of *B. abortus* OPS + 1 ug *B. melitensis* OPS-protein + 1 ug *B. suis* OPS together in 0.1 ml sterile saline as a single dose per mouse. Typically, for intra-peritoneal, sub-cutaneous and intra-muscular injections, the OPS were diluted in 0.1ml sterile saline, whereas for intra-nasal installation these were in 0.01 ml (half given into each nostril). Unless otherwise specified, mice were allowed to rest for 5 weeks before challenge.

Oral administration of the vaccine was proven to be effective in the case of *B. abortus* OPS vaccine to swine in Venezuela (US Patent No. 5,951,987). While the Applicant did not test oral administration of the new vaccine formulations of the present invention, it is suggested that efficacy of these vaccines do extend to oral administration.

### Challenges:

For intra-peritoneal challenge, a culture of *Brucella* was diluted serially in 9 ml sterile saline blanks and 5×10⁵ bacteria (as confirmed by plating) in 0.1 ml of sterile saline was given to each mouse. The mice were allowed to rest for 1 week before being sacrificed and assessed. Past studies suggested that infection by intra-nasal inoculation was more difficult to take place (Cherwonogrodzky J.W., and Di Ninno, V.L. 1994, *Brucella,* brucellosis, undulant fever, AB - Is it a threat? A review in question and answer form (U), Suffield Memorandum 1434, Defence Research Establishment Suffield, UNCLASSIFIED, page 6). Therefore, for intra-nasal challenge, 0.010 ml of a culture broth at an early phase of growth (about 5×10⁷bacteria/0.010 ml) was used without dilution and the mice were allowed to rest for 2 weeks rather than 1 week before being sacrificed and assessed.

### Assessment of Infection:

Mice seldom show any symptoms when infected with *Brucella,* although with the more serious strains they may show ruffled, grayish looking fur. Hence the only way to assess infection, was to weigh each mouse, sacrifice these by cervical dislocation and remove organs such as spleens for weighing and obtaining bacterial counts. In this case, the spleens were weighed for the ratio spleen wt/body wt., then crushed in 1 ml sterile saline by hand with a glass tissue grinder (Wheaton, 2 ml volume). This suspension was removed, another 1ml sterile saline was added to the chamber and crushing continued to complete the task and to rinse the inside of the chamber. This second 1 ml, was pooled with the first. To prevent possible aerosol generation, the work was performed inside a Biosafety 2a or 2b Cabinet inside a Biocontainment Level 3 (BL-3) area, and the investigator wore a seam sealed positive pressure hood (3M), HEPA filter with a blower powered by a battery pack, a sealed Tyvek overall, double gloves and boots. Five tissue grinders were used for each group of mice and these were sterilized between groups. Each tissue grinder had the chamber filled with 70% ethanol and the grinding handle inserted therein. The chamber was topped up with 70% ethanol, sprayed with the ethanol to decontaminate the outside and then allowed to sit for 30 minutes. Thereafter, the ethanol was poured out, the top of the chamber and the grinding handle wiped with a KimWipe^{TM} soaked in 70% ethanol and the grinding handle allowed to air dry. The ethanol was removed from the chamber with a sterile pipette and the chamber rinsed with sterile saline. Any adhering liquid was removed with another sterile pipette. For the crushed spleen in 2 ml of saline noted above, 0.1 ml of this was plated onto a plate of *Brucella* agar with 1.5 ppm of crystal violet, and 1 ml was transferred to a 9 ml sterile saline blank and dilutions with plating were repeated for these. Plates were incubated for 2 hours at 37°C, 5% CO₂ and 90% humidity and the CFU counted after one week incubation.

### Separation of blood components:

In instances where only serum was required, mice were given a double dose of 1:14 diluted Somnitol^{TM} (pentabarbitol), in 0.5 ml per mouse. Once anesthetized, a heart puncture was done with a 1ml syringe fitted with a 26-gauge needle, and whole blood was removed. Mice did not recover from the amount of anaesthetic given. The blood was transferred to a 1.5 ml Eppendorf™ microcentrifuge tube and the tube was left in the refrigerator at 4°C for a few hours to clot. It was then vortexed briefly to loosen the clot and centrifuged at 2000 X g for 5 minutes at room temperature or at 22°C to separate the serum, which formed the top layer, from blood cells. The serum was removed with a Pasteur pipette, pooled with serum from other mice in the group, and filtered through a 0.2 um filter. It was used within a few hours of preparation.

To fractionate serum into different molecular weight groups, whole serum (about 10 ml from 20 vaccinated mice) was placed initially into a 1000 molecular weight (m.w.) cutoff dialysis bag, and dialyzed in a 100 ml graduated cylinder against distilled water with magnetic stirring within a 4°C refrigerator. After 24 hours, the dialysate (1000 or less m.w. components), which remained in the cylinder, was frozen and freeze-dried. The serum was transferred to a 12,000 m.w. cutoff dialysis bag and dialyzed as before. This dialysate was 1,000-12,000 m.w. and freeze-dried. The serum within the dialysis bag had 12,000 or greater m.w. components and was freeze-dried.

To separate blood components, initially 1 ml of sterile saline was added to a 10 ml blood collection tube with heparin (10 fold heparin). One-tenth ml of 10-fold heparin was drawn into the 1ml syringe used to collect mouse blood. Whole blood (2 ml) was layered onto an equal volume of Lymphoprep^{TM} (Accurate Chemical and Scientific Corporation, Westbury, NY, USA) and centrifuged 1000 X g for 30 minutes at room temperature. The top layer was serum which was drawn off with a Pasteur pipette and then filtered through a 0.2 µm filter (final volume was about 1.5 ml). Mononuclear and polymorphonuclear cells formed 2 bands within the dextran solution. These were both drawn by a Pasteur pipette and washed twice with sterile saline (diluted in 0.85% sterile saline) and centrifuged 2000 X g for 30 min at room temperature. The supernatant layer was discarded and the cell pellet resuspended in 5 ml sterile saline, and washed as before and the cell pellet was resuspended in 1 ml sterile saline. The red blood cell pellet was removed, washed with sterile saline and resuspended in 1.5 ml of sterile saline.

The averages and standard error about the mean were calculated using the GraphPad Instat program (version 1.14).

### RESULTS AND DISCUSSION

### Example 1

### Cross-protection study

In this study, female balb/c mice were injected intra-peritoneally (i.p.) with either 0.1 ml sterile saline or *B. abortus* OPS vaccine/0.1 ml sterile saline. The mice were challenged 5 weeks later with 5×10^{5(delete4)} bacteria/0.1 ml sterile saline. They were sacrificed and assessed one week later. The results are shown in Table 1.

**Table 1:**

| Cross-protection study using a previous vaccine formulation | | |
|---|---|---|
| Group of mice (5 mice/group) | Spleen size | Average number of bacteria (CFU)/spleen |
| Control: mice infected with *B. abortus* 2308 | Normal | 13,200 ± 4,170 |
| Mice vaccinated with 1 ug OPS, infected with *B* . *abortus* 2308 | Normal | 5,960 ± 2,440 |
| Mice vaccinated with 100 ug OPS, infected with *B. abortus* 2308 | Normal | 164,000 ± 95,100 |
| | | |
| Control: mice infected with *B. suis* 145 | Large | 4,460,000 ± 454,000 |
| Mice vacinaed with 1 ug OPS, in- | Normal | 908,000 ± 719,000 |
| fected with B. *suis* 145 | | |
| Mice vaccinated with 100 ug OPS, infected with *B. suis* 145 | Normal | 511,000 ± 246,000 |
| | | |
| Control: mice infected with *B. melitensis* 16M | Large | 279,000 ± 36,800 |
| Mice vaccinated with 1 ug OPS, infected with *B. melitensis* 16M | Normal | 325,000 ± 183,000 |
| Mice vaccinated with 100 ug OPS, infected with *B. melitensis* 16M | Normal | 102,200 ± 18,000 |

By convention, minimal protection is when vaccinates have 10-fold less bacteria in their spleens than unvaccinated controls. The above shows that the former vaccine formulation as disclosed in US 5,951,987 was not protective against *B. melitensis* 16M nor *B. suis* 145. It was also of little effect against *B. abortus* 2308, an unusual strain of *B. abortus* that sometimes changes its antigens to evade the immune system of the mouse. As the previous vaccine, formulated from *B. abortus* 1119-3 (that expresses the "A" OPS) was not protective against *B. melitensis* 16M (that expresses "M" OPS), *B. suis* 145 (that expresses "M" as well as "A" OPS) nor in this case *B. abortus* 2308 (which is variable and sometimes shifts from "A" to "M" OPS), it was apparent that the previous vaccine (as disclosed in US 5,951,987) was limited in its scope of protection. At the time of the previous patent submission, in which the vaccine formulated from *B. abortus* 1119-3 was protective against the species and strains of *Brucella* tested, this was unobvious. The previous patent and past publication taught away from the new finding that a combination vaccine, one with more than one OPS, was needed for wider protection against *Brucella.*

As noted in the above table, large doses *of B. abortus* 1119-3 OPS (i.e. 100 ug/mouse) did not offer any advantage for protection and indeed appeared to be counter-protective from *B. abortus* 2308 challenge. The Applicant has observed that low doses are more effective than high doses, one dose is better than three doses, and formulations that enhance antibody production (e.g. OPS on lipopolysaccharide, OPS in liposomes) are counter-productive. Antibody induction is counter-productive because antibody will opsonize, or coat, invading bacteria, these are recognized by white blood cells which ingest the bacterium, and then the parasitic bacterium is inside the host white blood cell, its preferred environment. In contrast, low doses of OPS vaccine appear to stimulate cell-mediated responses.

### Example 2

### Effectiveness of various combination vaccine candidates on vaccinated mice infected one day after vaccination

**Table 2:**

| | | |
|---|---|---|
| Response of vaccinated mice infected one day after vaccination. Median bacterial numbers are shown in brackets. Each group represents five mice. The animals were sacrificec seven days after infection. | | |

| Vaccine given to group | Spleen wt/body wt | Bacterial CFU/spleen |
|---|---|---|
| None, saline control | 0.0211 ± 0.0028 | 2,400,000 ± 250,000 (2,200,000) |
| 1 ug *B. melitensis* OPS | 0.0163 ± 0.0027 | 1,690,000 ± 451,000 (1,860,000) |
| 100 ug *B. melitensis* OPS | 0.0114 ± 0.0007 | 850,000 ± 361,000 (880,000) |
| 0.5 ug *B. melitensis* OPS + 0.5 ug *B. ab- ortus OPS* | 0.0172 ± 0.0012 | 1,820,000 ± 381,000 (2,140,000) |
| 50 ug *B. melitensis* OPS + 50 ug *B. abor- tus* | 0.0129 ± 0.0015 | 984,000 ± 386,000 (680,000) |
| **0.5 ug *B. melitensis* OPS-protein + 0.5 ug *B. abortus* OPS** | **0.0089 ± 0.0003** | **624,000 ± 359,000 (96,000)** |
| 50 ug *B. melitensis* OPS-protein + 50 ug *B. abortus* OPS | 0.0183 ± 0.0015 | 1,550,000 ± 351,000 (1,560,000) |

Throughout several years of studying *Brucella* infections in mice, it was evident that not all infected mice come down with brucellosis. Usually about 5-10% of the mice are infected, as evidenced by *Brucella* in their spleens, but as the numbers are trivial, they are obviously not developing brucellosis. In the publication by Detilleux et al. (Detilleux, P.G., Deyoe, B.L., and Cheville, N.F. 1990. Penetration and Intracellular Growth of *Brucella abortus* in nonphagocytic cells in vitro. *Infection and Immunity* 58: 2320-2328) it was ob*ortus* in nonphagocytic cells in vitro. *Infection and Immunity* 58: 2320-2328) it was observed that *Brucella* takes about 2 hours to infect mammalian cells. The Applicant has also observed over the years that the most virulent forms of *Brucella* (e.g. *B. melitensis* 16M) shed their OPS, potentially vaccinating the experimental animal before infection. Two questions were asked. Does a combination vaccine protect against more strains and serotypes of *Brucella?* Does this vaccine work in a very short time frame, such as a day instead of several weeks?

In the above data, initially it appears that none of the vaccine formulations were protective when given a day before infection. That is because a single mouse in a group of 5 that either was not responsive to the vaccine or if the vaccine was not properly administered, will have high bacterial counts that will skew the average. As the data was entered, it was obvious that the one highlighted (0.5 ug *B. melitensis* OPS-protein complex + 0.5 ug B. abortus OPS) was actually very protective. In this instance, the median (or the middle number) was more reflective than the average bacterial count. It can also be seen that the vaccine does protect mice against *B. melitensis* 16M infection, even when given as early as a day before infection (and other studies in the Applicant's laboratory at the Defence Research Establishment Suffield (DRES) showed vaccine protection in as little as 1 hour before infection). The potential is that livestock being shipped to an area of high brucellosis prevalence, or a traveller or soldier travelling to a country where *Brucella* is endemic, can be vaccinated with protection developing as they are en route.

### Example 3

### Effectiveness of various combination vaccine candidates on vaccinated mice infected five weeks after vaccination.

**Table 3:**

| | | |
|---|---|---|
| Response of vaccinated mice infected five weeks after vaccination. Median bacteria numbers are shown in brackets. Each group represents five mice. The animals were sacrifice seven days after infection. | | |

| Vaccine given to group | Spleen wt/body wt | Bacterial CFU/spleen |
|---|---|---|
| None, saline control | 0.0117 ± 0.0015 | 1,120,000 ± 195,000 (1,300,000) |
| 1 ug *B. melitensis* OPS | 0.0066 ± 0.0004 | 48,500 ± 23,000 (44,000) |
| 100 ug *B. melitensis* OPS | 0.0073 ± 0.0002 | 365,000 ± 167,000 (290,000) |
| 0.5 ug *B. melitensis* OPS + 0.5 ug *B. ab- ortus* OPS | 0.0061 ± 0.0004 | 35,800 ± 6,460 (42,000) |
| 50 ug *B. melitensis* OPS + 50 ug *B. abor- tus* | 0.0065 ± 0.0005 | 162,000 ± 70,000 (150,000) |
| 0.5 ug *B. melitensis* OPS-protein + 0.5 ug *B. abortus* OPS | 0.0059 ± 0.0003 | 67,600 ± 30,200 (92,000) |
| 50 ug *B. melitensis* OPS-protein + 50 ug *B. abortus* OPS | 0.0069 ± 0.0004 | 85,100 ± 26,000 (70,000) |

The results shown in Tables 2 and 3 above, demonstrate that overall, 0.5 ug *B. melitensis* OPS-protein + *B. abortus* OPS was most effective in providing protection against *B. melitensis* 16M infection, either one day or five weeks after vaccination. Higher dose of the same vaccine does not seem to be as effective, probably because it elicit antibodies. Tables 2 and 3 also show that given several weeks, OPS vaccines are effective for protecting mice from *B. melitensis* 16M infections.

### Example 4

### Effectiveness of different vaccine candidates against different species and strains of Brucella infections

In this case, Balb/c mice were vaccinated by the intra-peritoneal route with various vaccine candidates. The mice were challenged (i.p.) four weeks later with different species of *Brucella.* One week after infection, the mice were sacrificed and assessed for brucellosis. Each group represents the average for five mice, each mouse was given 1 ug of each OPS.

**Table 4:**

| Effect of various vaccines against different type of *Brucella* infections. | | | | |
|---|---|---|---|---|
| | Spleen wt/body wt (first line) Bacterial count (CFU) in spleen (second and third line) | | | |
| | *B. melitensis* 16M infection | *B. suis* 145 infection | *B. abortus* 30 infection | *B. abortus* 2308 infection |
| Control - no vaccine | 0.0162 ± 0.0025 | 0.0052 ± 0.0008 | 0.0080 ± 0.0008 | 0.0047 ± 0.0004 |
| | 3,470,000 ± 740,000 | 346,000 ± 74,900 | 970,000 ± 191,000 | 155,000 ± 74,300 |
| *B. abortus* OPS vaccine | 0.0077 ± 0.0012 | 0.0052 ± 0.0003 | 0.0077 ± 0.0014 | 0.0055 ± 0.0010 |
| | 270,000 ± 136,000 | 91,200 ± 20,500 | 391,000 ± 146,000 | 7,740 ± 4,030 |
| ***B. suis* OPS vaccine** | **0.0055 ± 0.0003** | **0.0045 ± 0.0003** | **0.0048 ± 0.0005** | **0.0050 ± 0.0005** |
| | **6,650 ± 2,310** | **20 ± 11** | **148 ± 91** | **9,920 ± 5,090** |
| *B. melitensis OPS-protein* | 0.0059 ± 0.0006 | 0.0045 ± 0.0004 | 0.0051 ± 0.0004 | 0,0065 ± 0.0008 |
| | 25,900 ± 13,800 | 0 ± 0 | 108 ± 59 | 238,000 ± 77,400 |
| *B. abortus* OPS + B. melitensis OPS-protein | 0.0055 ± 0.0004 | 0.0052 ± 0.0005 | 0.0045 ± 0.0006 | 0.0053 ± 0.0005 |
| | 36,900 ±16,400 | 12 ± 5 | 212 ± 212 | 112,000 ± 63,300 |
| *B. abortus* OPS + B. melitensis OPS-protein + B. *suis* OPS | 0.0037 ± 0.0002 | 0.0048 ± 0.0004 | 0.0050 ± 0.0003 | 0.0052 ± 0.0004 |
| | (no water 1 day) | 1 ± 1 | 56 ± 51 | 84,900 ± 32,600 |
| | 44,700 ± 31,400 | | | |

The above results show that either a combination of OPS and OPS-protein antigens from different *Brucellae,* or an OPS preparation from a single strain of *Brucella,* (for example *B. suis* 145) that expresses more than one OPS, were effective in protecting mice from brucellosis. The most remarkable of the vaccine "cocktails" tested is that of *B. suis* 145 OPS. It not only protects against a very wide range of *Brucella* species, but it also appears to work the best for each. It is believed that this greater OPS vaccine protection is because *B. suis* 145 not only expresses both "A" and "M" (instead of just one) OPS, but that it also expresses one (which Dr. Brad Berger at DRES has isolated) or more additional and previously unknown OPS.

It is also interesting to note that the vaccines work best (i.e. there is a greater contrast between non-vaccinated control mice and vaccinated mice) for the species and strains of *Brucellae* that are the most virulent (i.e. species or strains of *Brucella* that cause a large spleen sizes and high bacterial numbers in the spleens of unvaccinated animals). OPS is on the surface of smooth *Brucella* and it is also shed by the most virulent species and strains of *Brucella.* It is now believed that the OPS is a virulence factor that reduces the resistance of the host. By vaccinating with OPS and inducing an immunity against this component, it is likely that the host has an immunity both against the *Brucella* bacterium but also against the OPS that they shed.

### Example 5

### Effectiveness of different vaccine candidates using various administration routes

In this case, mice were vaccinated by different routes and various vaccines candidates were used. The mice were challenged four weeks after vaccination with different species of *Brucella* and were sacrificed and assessed one week after vaccination. Each group represents the average of 15 mice and each mouse was given 1 ug of OPS.

**Table 5:**

| Effectiveness of different vaccine candidates using various administration of vaccine routes. | | | | |
|---|---|---|---|---|
| | Spleen wt/body wt Bacterial count (CFU) in spleen | | | |
| | *B. melitensis* 16M challenge, i.n. | *B. suis* 145 challenge, i.n. | *B. abortus* 30 challenge, i.n. | *B. abortus* 2308 challenge, i.n |
| Saline control, i.m. | 0.0082 ± 0.0004 | 0.0117 ± 0.0010 | 0.0047 ± 0.0001 | 0.0052 ± 0.0001 |
| | 124,000 ± 34,900 | 448,000 ± 122,000 | 87 ± 24 | 7,040 ± 2,540 |
| *B. suis* OPS, i.n. | 0.0088 ± 0.0004 | | | |
| | 288,000 ± 90,100 | | | |
| *B.* suis OPS, i.m. | 0.0071 ± 0.0004 | 0.0082 ± 0.0006 | 0.0045 ± 0.0001 | 0.0048 ± 0.0001 |
| | 288,000 ± 90,100 | 48,500 ± 14,900 | 65 ± 27 | 954 ± 523 |
| *B. abortus* OPS + *B. melitensis*OPS-protein + *B. suis* OPS, i.m. | 0.0065 ± 0.0002 | 0.0067 ± 0.0005 | 0.0045 ± 0.0001 | 0.0050 ± 0.0003 |
| | 22,000 ± 10,600 | 31,400 ± 11,600 | 20 ± 7 | 125 ± 68 |
| Saline control, s.c. | 0.0074 ± 0.0004 | 0.01127 ± 0.0010 | 0.0049 ± 0.0001 | 0.0052 ± 0.0001 |
| | 62,000 ± 21,900 | 339,000 ± 94,400 | 230 ± 99 | 6,690 ± 2,040 |
| *B. suis* OPS, s.c. | 0.0060 ± 0.0005 | 0.0072 ± 0.0005 | 0.0046 ± 0.0002 | 0.0050 ± 0.0001 |
| | 15,900 ± 12,000 | 29,000 ± 7,680 | 53 ± 19 | 361 ± 179 |
| *B. abortus* OPS + *B. melitensis*OPS-protein + B.*suis* OPS, s.c. | 0.0064 ± 0.0002 | 0.0070 ± 0.0004 | 0.0047 ± 0.0001 | 0.0047 ± 0.0001 |
| | 7,100 ± 1 980 | 25,000 ± 6,000 | 31 ± 20 | 154 ± 65 |

The above shows that either a "cocktail" vaccine, made by adding purified antigens from different bacteria or by adding different antigens prepared from one bacterium, given by different routes can protect mice from a wide range of different *Brucella* species and strains.

Recently the *B. suis* 145 OPS vaccine (1 ug/mouse) was given to anaesthetized mice (female, balb/c) by the intra-nasal route and mice were challenged 4 weeks later with *B. suis* 145 also given intra-nasally. Administration by this route did not appear to offer any protection. At the Applicant's research establishment, it has been observed that vaccination against other infectious bacteria by the intranasal route appears to induce antibodies in the respiratory tract. The induction of antibodies is counter-productive for vaccine protection against *Brucella.* Recently it has been found that *B. suis* 145 OPS offers protection in mice from brucellosis when given in doses ranging from 1 ng to 100 ug. It was not protective when doses were less than a nanogram. It is likely that this vaccine can be effective for protecting mice from brucellosis when given intranasally, but that the lower doses, in the nanogram rather than microgram range, must be used to induce a cell-mediated response and to avoid a counter-productive antibody response.

### Example 6

### Passive Immunity

Mice were vaccinated with 1 ug *B. suis* OPS intraperitoneally. An hour later these were sacrificed and their serum collected. Half a ml of serum was given to each naïve mouse about 3 hours later. Recipient mice were challenged with *B. melitensis* 16M an hour after receiving the noted serum. The results are shown in Table 6.

**Table 6:**

| Passive Immunity | | | | |
|---|---|---|---|---|
| Group | Mouse | Mouse wt. (g) | Spleen wt (g) | Bacteria in spleen |
| CONTROL - mice received 0.5 ml of serum from unvaccinated mice. Infected 1 hr later, assessed 1 week later | 1 | 23.52 | 0.4034 | 5,370,000 |
| | 2 | 21.28 | 0.4682 | 4,280,000 |
| | 3 | 22.24 | 0.4491 | 2,000,000 |
| | 4 | 20.56 | 0.4027 | 1,860,000 |
| | 5 | 23.36 | 0.4618 | 2,580,000 |
| TEST - mice received 0.5 ml of serum from vaccinated mice Infected 1 hr later, assessed 1 week later. | 1 | 21.82 | 0.1174 | 620,000* |
| | 2 | 19.52 | 0.4663 | 260.000* |
| | 3 | 24.66 | 0.2162 | 1,000,000 |
| | 4 | 21.64 | 0.6244 | 0 |
| | 5 | 21.52 | 0.6765 | 2,360 |

| | | | | |
|---|---|---|---|---|
| * colonies were unusually small | | | | |

The above shows that protection against brucellosis is very rapid (protection occurs within 1 hour of being vaccinated) and that this protection can be transferred by giving immune serum to naive mice to give them protection as well.

### Example 7

### Passive Immunity (continued)

In a second passive immunity study, mice were vaccinated with 1 ug each with *B. melitensis* 16M OPS-protein given i.p, sacrificed four weeks later, and their blood was fractionated. Naïve mice (Table 7), were recipients (the number of mice used was small for this was proof of concept to justify further study). A control mouse was given saline intraperitoneally. For the other groups, these received (from the first set of sacrificed mice) their red blood cells (0.5 ml/mouse), serum (0.5 ml/mouse) or washed white blood cells (in 0.3 ml saline/mouse). Mice given these injections were challenged one day later with 5x10⁴ bacteria given intra-peritoneally, and were sacrificed and assessed one week later.

**Table 7:**

| Passive Immunity | | | | |
|---|---|---|---|---|
| Mice were given: | Mouse | Body wt (g) | Spleen wt. (g) | Bacteria in spleen |
| Saline | 1 | 37.44 | 0.7547 | 5,340,000 |
| Red blood cells | 1 | 37.52 | 0.4737 | 2,980,000 |
| | 2 | 39.18 | 0.8639 | 2,840,000 |
| | 3 | 37.12 | 0.7522 | 2,480,000 |
| White blood cells | 1 | 35.52 | 0.4611 | 740,000 |
| | 2 | 36.86 | 0.3960 | 1,860,000 |
| | 3 | 36.40 | 0.5360 | 1,000,000 |
| Serum | 1 | 39.66 | 0.4098 | 440,000 |
| | 2 | 35.06 | 0.3956 | 2,500,000 |
| | 3 | 37.92 | 0.2497 | 280,000 |

The above shows that in vaccinated mice, the protective factor appears to be made in white blood cells and then released into the serum. This protective factor is produced rapidly (within 1 hour as evidenced by Table 6), continues to be produced for several weeks (as evidenced by the above Table 7) and can be transferred to naive mice to offer them protection from brucellosis as well.

"The features disclosed in the foregoing description and in the claims may, both separately and in any combination thereof, be material for realising the invention in diverse forms thereof."

## Claims

1. A vaccine comprising a combination of outer-polysaccharides, extracted from *Brucella,* wherein said combination of outer-polysaccharides comprises "A" outer-polysaccharide and "M" outer-polysaccharide; and "R" protein antigens or a *Brucella* outer-polysaccharide-protein complex.

2. A vaccine as claimed in claim 1, wherein said extracted outer-polysaccharide is at least 90 percent pure.

3. A vaccine as claimed in claim 1, wherein said "A" outer-polysaccharide is extracted from *Brucella* species selected from the group consisting of *B. abortus* biovar 1, *B. abortus* biovar 2, *B. abortus* biovar 3, *B. abortus* biovar 6, *B. melitensis* biovar 2, *B. suis* biovar 1, *B. suis* biovar 2, *B. suis* biovar 3, *B. neotomae* and *B. maris;* said "M" outer-polysaccharide is extracted from *Brucella* species selected from the group consisting of *B. abortus* biovar *4, B. abortus* biovar 5, *B. abortus* biovar 9, *B. melitensis* biovar 1, *B. suis* biovar 5; and said "R" antigens are protein and/or core polysaccharide extracted from *Brucella* species selected from the group consisting of *B. ovis* and *B. canis.*

4. A vaccine as claimed in claim 1, wherein said "A" and "M" outer-polysaccharides are extracted from *Brucella* species selected from the group consisting of B. *abortus* biovar 7, *B. melitensis* biovar 3 and *B. suis* biovar 4, and said "R" antigens are extracted from *Brucella* species selected from the group consisting of *B. ovis* and *B. canis.*

5. A vaccine as claimed in claim 1, wherein said "A" outer-polysaccharide is extracted from *Brucella* species selected from the group consisting of *B. abortus* biovar 1, *B. abortus* biovar 2, *B. abortus* biovar 3, *B. abortus* biovar 6, *B. melitensis* biovar 2, *B. suis* biovar 1, *B. suis* biovar 2, *B. suis* biovar 3, *B. neotomae* and *B. maris;* said "M" outer-polysaccharide is extracted from *Brucella* species selected from the group consisting of *B. abortus* biovar 4, *B. abortus* biovar 5, *B. abortus* biovar 9, *B. melitensis* biovar 1, *B. suis* biovar 5; and said outer-polysaccharide-protein complex is selected from the group consisting of outer-polysaccharide and *Brucella* proteins selected from the group consisting of *Brucella* surface proteins, *Brucella* surface enzymes, *Brucella* membrane proteins, and *Brucella* cytoplasmic proteins.

6. A vaccine as claimed in claim 1, wherein said "A" and "M" outer-polysaccharides are extracted from *Brucella* species selected from the group consisting of *B. abortus* biovar 7, *B. melitensis* biovar 3 and *B. suis* biovar 4, and said outer-polysaccharide-protein complex is selected from the group consisting of outer-polysaccharide and *Brucella* proteins selected from the group consisting of *Brucella* surface proteins, *Brucella* surface enzymes, *Brucella* membrane proteins, and *Brucella* cytoplasmic proteins.

7. A vaccine as claimed in claim 1, wherein each of said outer-polysaccharide is in an amount of 1 ng to 100 µg for vaccination of mice.

8. A vaccine as claimed in claim 7, wherein said outer-polysaccharide is in an amount of 1 µg for vaccination of mice.

## Patentansprüche

1. Impfstoff, umfassend eine Kombination von Außen-Polysacchariden, extrahiert aus *Brucella,* wobei die Kombination von Außen-Polysacchariden "A" Außen-Polysaccharid und "M" Außen-Polysaccharid umfaßt; und "R" Proteinantigene oder einen *Brucella* Außen-Polysaccharid-Proteinkomplex.

2. Impfstoff nach Anspruch 1, wobei das extrahierte Außen-Polysaccharid zu mindestens 90 Prozent rein ist.

3. Impfstoff nach Anspruch 1, wobei das "A" Außen-Polysaccharid von *Brucella* Spezies ausgewählt aus der Gruppe bestehend aus *B. abortus* biovar 1, *B. abortus* biovar 2, B. *abortus* biovar 3, *B. abortus* biovar 6, *B. melitensis* biovar 2, *B. suis* biovar 1, *B. suis* biovar 2, *B. suis* biovar 3, *B. neotomae* und *B. maris* extrahiert ist: das "M" Außen-Polysaccharid von *Brucella* Spezies ausgewählt aus der Gruppe bestehend aus *B. abortus* biovar 4, *B. abortus* biovar 5, *B. abortus* biovar 9, *B. melitensis* biovar 1, *B. suis* biovar 5 extrahiert ist; und die "R" Antigene Proteine und/oder Kern-Polysaccharide sind, die von *Brucella* Spezies ausgewählt aus der Gruppe bestehend aus *B. ovis* und *B. canis* extrahiert sind.

4. Impfstoff nach Anspruch 1, wobei das "A" und "M" Außen-Polysaccharide aus *Brucella* Spezies ausgewählt aus der Gruppe bestehend aus *B. abortus* biovar 7, *B. melitensis* biovar 3 und *B. suis* biovar 4 extrahiert sind, und die "R" Antigene von *Brucella* Spezies ausgewählt aus der Gruppe bestehend aus *B. ovis* und *B. canis* extrahiert sind.

5. Impfstoff nach Anspruch I, wobei das "A" Außen-Polysaccharid aus *Brucella* Spezies ausgewählt aus der Gruppe bestehend aus *B. abortus* biovar 1, *B. abortus* biovar 2, B. *abortus* biovar 3, B. *abortus* biovar 6, *B. melitensis* biovar 2, *B. suis* biovar 1, *B. suis* biovar 2, *B. suis* biovar 3, *B. neotomae* und *B. maris* extrahiert ist, das "M" Außen-Polysaccharid aus *Brucella* Spezies ausgewählt aus der Gruppe bestehend aus *B. abortus* biovar 4, B. *abortus* biovar 5, *B. abortus* biovar 9, *B. melitensis* biovar 1, B. *suis* biovar 5 extrahiert ist; und der Außen-Polysaccharid-Proteinkomplex ausgewählt ist aus der Gruppe bestehend aus Außen-Polysaccharid und *Brucella* Proteinen, ausgewählt aus der Gruppe bestehend aus *Brucella* Oberflächenproteinen, *Brucella* Oberflächenenzymen, *Brucella* Membranproteinen, *Brucella* Oberflächenproteinen und *Brucella* cytoplasmatischen Proteinen.

6. Impfstoff nach Anspruch 1, wobei die "A" und "M" Außen-Polysaccharide aus *Brucella* Spezies ausgewählt aus der Gruppe bestehend aus *B. abortus* biovar 7, *B. melitensis* biovar 3 und *B. suis* biovar 4 extrahiert sind, und der Außen-Polysaccharid-Proteinkomplex ausgewählt ist aus der Gruppe bestehend aus Außen-Polysaccharid und *Brucella* Proteinen ausgewählt aus der Gruppe bestehend aus *Brucella* Oberflächenproteinen, *Brucella* Oberflächenenzymen, *Brucella* Membranproteinen, *Brucella* Oberflächenproteinen, und *Brucella* cytoplasmatischen Proteinen.

7. Impfstoff nach Anspruch 1, wobei jedes der Außen-Polysaccharide in einer Menge von 1 ng bis 100 µg für Mäuse vorliegt.

8. Impfstoff nach Anspruch 7, wobei das Außen-Polysaccharid in einer Menge von 1 µg für Mäuse vorliegt.

## Revendications

1. Vaccin comprenant une combinaison de polysaccharides extérieurs, extraits de Brucella, où ladite combinaison de polysaccharides extérieurs comprend un polysaccharide extérieur "A" et un polysaccharide extérieur "M", et des protéines antigènes "R" ou un complexe polysaccharide extérieur de Brucella / protéine.

2. Vaccin selon la revendication 1, dans lequel ledit polysaccharide extérieur extrait est pur à au moins 90 %.

3. Vaccin selon la revendication 1, dans lequel ledit polysaccharide extérieur "A" est extrait d'une espèce de Brucella choisie dans le groupe constitué par B. abortus biovar 1, B. abortus biovar 2, B. abortus biovar 3, B. abortus biovar 6, B. melitensis biovar 2, B. suis biovar 1, B. suis biovar 2, B. suis biovar 3, B. neotomae et B. maris ; ledit polysaccharide extérieur "M" est extrait d'une espèce de Brucella choisie dans le groupe constitué par B. abortus biovar 4, B. abortus biovar 5, B. abortus biovar 9, B. melitensis biovar 1, B. suis biovar 5 ; et lesdits antigènes "R" sont des protéines et/ou un polysaccharide de coeur extrait d'une espèce de Brucella choisie dans le groupe constitué par B. ovis et B. canis.

4. Vaccin selon la revendication 1, dans lequel lesdits polysaccharides extérieurs "A" et "M" sont extraits d'une espèce de Brucella choisie dans le groupe constitué par B. abortus biovar 7, B. melitensis biovar 3 et B. suis biovar 4, et lesdits antigènes "R" sont extraits d'une espèce de Brucella choisie dans le groupe constitué par B. ovis et B. canis.

5. Vaccin selon la revendication 1, dans lequel ledit polysaccharide extérieur "A" est extrait d'une espèce de Brucella choisie dans le groupe constitué par B. abortus biovar 1, B. abortus biovar 2, B. abortus biovar 3, B. abortus biovar 6, B. melitensis biovar 2, B. suis biovar 1, B. suis biovar 2, B. suis biovar 3, B. neotomae et B. maris ; ledit polysaccharide extérieur "M" est extrait d'une espèce de Brucella choisie dans le groupe constitué par B. abortus biovar 4, B. abortus biovar 5, B. abortus biovar 9, B. melitensis biovar 1, B. suis biovar 5 ; et ledit complexe de polysaccharide extérieur / protéine est choisi dans le groupe constitué par un polysaccharide extérieur et des protéines de Brucella choisies dans le groupe constitué par les protéines de surface de Brucella, les enzymes de surface de Brucella, les protéines de membrane de Brucella, et les protéines cytoplasmiques de Brucella.

6. Vaccin selon la revendication 1, dans lequel lesdits polysaccharides extérieurs "A" et "M" sont extraits d'une espèce de Brucella choisie dans le groupe constitué par B. abortus biovar 7, B. melitensis biovar 3 et B. suis biovar 4, et ledit complexe de polysaccharide extérieur / protéine est choisi dans le groupe constitué par un polysaccharide extérieur et des protéines de Brucella choisies dans le groupe constitué par les protéines de surface de Brucella, les enzymes de surface de Brucella, les protéines de membrane de Brucella, et les protéines cytoplasmiques de Brucella.

7. Vaccin selon la revendication 1, dans lequel chacun desdits polysaccharides extérieurs est présent en une quantité de 1 ng à 100 µg pour la vaccination de souris.

8. Vaccin selon la revendication 7, dans lequel ledit polysaccharide extérieur est présent en une quantité de 1 µg pour la vaccination de souris.
